# EUROPEAN PATENT APPLICATION

(11) **EP 4 826 647 A1**
(43) Date of publication of application: **21.10.2026**
(21) Application number: 25171310.3
(22) Date of filing: 17.04.2025
(51) Int. Cl.: G06T 7/00, G06T 7/11

(54) **LANGUAGE-MODEL ASSISTED ABNORMALITY DETECTION IN 3D MEDICAL IMAGE DATA**

(71) Applicant: Siemens Healthineers AG, 91301 Forchheim (DE)
(72) Inventor: SHINAGAWA, Yoshihisa, Downingtown, 19335 (US); SAHIN, Mehmet Berk, West Lafayette, 47906 (US); YEREBAKAN, Halid, Carmel, 46033 (US); FARHAND, Sepehr, Malvern, 19355 (US); ABDISHEKTAEI, Mohammad, Charlottesville, 22903 (US); HERMOSILLO VALADEZ, Gerardo, West Chester, 19382 (US)
(74) Representative: Kraus & Lederer PartGmbB

(57) **Abstract**

The invention relates to enabling detection of abnormalities represented in 3D medical image data. For this purpose, a method may comprise receiving training input data comprising 3D medical image data and medical reports associated with the 3D medical image data. Further, the method may comprise applying a trained language model (510) for extracting anatomy phrases from the medical reports. Further, the method may comprise training a classification model (550) based on the 3D medical image data and the anatomy phrases, whereby the classification model (550) is trained to generate output data indicative of an abnormality represented in the 3D medical image data.

## Description

Independent of the grammatical term usage, individuals with male, female or other gender identities are included within the term.

### Technical field

The present disclosure relates to abnormality detection in three-dimensional (3D) medical image data.

### Background

Analysis of 3D medical image data, such as 3Dimage data obtained by magnetic-resonance imaging (MRI) or by computed tomography (CT), is a complex and challenging task. This specifically holds true if the 3D medical image data cover larger parts of the patient's body. For example, the 3D medical image data could be based on a whole-body scan of the patient, or on a scan of a major body part, such as the head or thorax. As a result, the 3D medical image data may cover a relatively high number of anatomical details.

To facilitate handling of medical image data, a Computer-Aided Detection (CAD) system can be used. Such a system may detect abnormalities in the medical image data, e.g., image structures corresponding to diseased tissue, in an automated or semi-automated manner, utilizing machine-learning (ML) / Artificial Intelligence (Al) mechanisms. Based on the output of such CAD system, the image data (or parts thereof) can for example be routed images to different radiologists with different subspecialties, or further scans to collect supplemental image data may be initiated. However, implementation of such CAD system becomes increasingly difficult as the volume covered by 3D medical image data increases. Implementation of a whole-body CAD system thus constitutes a challenging task.

Convolutional Neural Networks (CNNs) can learn and extract meaningful features from complex medical images and are widely used in medical imaging diagnosis. For example, existing CNNs and learning strategies may be adopted for medical image classification tasks.

For example, "Alzheimer's disease classification: a comprehensive study" by Assmi, A., et al., Multimed Tools Appl 83, 70193-70216, https://doi.org/10.1007/s11042-024-18306-9 (2024), compares classification performance of different pre-trained networks for Alzheimer's disease detection on brain MRIs.

Further, "Long-Tailed Classification of Thorax Diseases on Chest X-Ray: A New Benchmark Study", by Holste, G., et al., Data Augment Label Imperferctions, doi: 10.1007/978-3-031-17027-0_3 (2022), describes classification of thorax diseases on chest X-rays utilizing an architecture denoted as "ResNet".

A still further approach is to utilize Vision Transformers (ViTs) and divide images into separate patches and process them as a sequence with an attention mechanism, as for example described in "Attention Is All You Need", by Vaswani, A., et al., http://arxiv.org/abs/1706.03762 (2023). Thanks to their capacity to capture long-range dependencies and adapt to images of varying sizes ViTs are widely used for medical image classification. ViT architectures applied in the medical field include Swin Transformer, Twins, and MViT, see "MedMamba: Vision Mamba for Medical Image Classification", by Yue, Y. and Li, Z., http://arxiv.org/abs/2403.03849 (2024). ViTs may also be utilized in modalities other than MRI for diagnosis.

"A visual-language foundation model for pathology image analysis using medical twitter", by Huang, Z. et al., Nature medicine, 29(9):2307-2316 (2023) describes a multimodal artificial intelligence with both image and text understanding, denoted as PLIP (pathology language-image pretraining) for use in computational pathology.

A model denoted as "MedSAM" was proposed to segment everything in medical images, see "Segment anything in medical images", by Ma J., et al., Nat Commun 15, 654, https://doi.org/10.1038/s41467-024-44824-z (2024).

A model denoted as "RETGround" was proposed to detect and diagnose different diseases in the field of ophthalmology, see "A foundation model for generalizable disease detection from retinal images", by Zhou, Y. et al., Nature, 622(7981):156-163, 2023.

A state space sequence model (SSM) was proposed to describe dynamic systems and model long sequences but was challenging to train due to high computational and memory demands. The Structured State Space Sequence Model (S4) overcomes these limitations by using Normal Plus Low-Rank (NPLR) representation and Woodbury identity for efficient matrix inversion, speeding up the convolution kernel computation. However, the time-invariant nature of S4 limits its ability to learn context-based reasoning ability. Mamba solved this problem by merging time-varying parameters into SSM to learn context-dependent information, see "Mamba: Linear-time sequence modeling with selective state spaces", by Gu, A. and Dao, T., arXiv: 2312.00752 (2023).

An architecture denoted as "VMamba" was introduced as a vision backbone that utilizes cross-scan module to apply Mamba effectively in image domain, see "Vmamba: Visual state space model", by Liu, Y., et al., arXiv:2401.10166 (2024).

Because of its good performance in visual tasks, researchers proposed variants of the Mamba architecture for disease classification in medical imaging, such as the above-mentioned MedMamba architecture, which may be applied for two-dimensional (2D) generalized disease classification. The MedMamba architecture introduced a processing block denoted as "SS-Conv-SSM" block, which combines a CNN and Mamba, to learn local features as well as the long-range dependencies. Further, an architecture denoted as "nnMamba" (Mamba-In-Convolution with Channel-Spatial Siamese learning block) was proposed to model long-range relationships of voxels in 3D medical images, see "nnMamba: 3D Biomedical Image Segmentation, Classification and Landmark Detection with State Space Model", by Gong, H., et al., http://arxiv.org/abs/2402.03526 (2024).

Some studies proposed algorithms for disease classification guided by radiology reports. For example, an algorithm denoted as "ReportGuidedNet" uses structured radiology reports generated from unstructured reports by Natural Language Processing (NLP) together with images, see "Boosting Deep Learning for Interpretable Brain MRI Lesion Detection through the Integration of Radiology Report Information" by Dai, L., et al., Radiol Artif Intell., doi: 10.1148/ryai.230520 (2024). An algorithm denoted as "AGXNet" involves transforming a radiology report to matrices whose rows and columns correspond to anatomical landmarks and disease names, respectively. AGXNet consists of two CNNs to estimate the rows and columns separately and they leveraged anatomical landmarks to improve the disease classification performance on chest X-ray images, see "Anatomy-Guided Weakly-Supervised Abnormality Localization in Chest X-rays", by Yu, K., et al., http://arxiv.org/abs/2206.12704 (2022).

An architecture denoted as "Anatomy-XNet" was proposed as an anatomy-aware attention-based thoracic disease classification network and utilizes anatomy-aware attention mechanisms to focus on spatial features based on pre-identified anatomical regions, see "Anatomy-XNet: An Anatomy Aware Convolutional Neural Network for Thoracic Disease Classification in Chest X-Rays", by Kamal, U., et al., IEEE Journal of Biomedical and Health Informatics, 26(11), 5518-5528. doi:10.1109/jbhi.2022.3199594 (2022).

In view of the above, there is a need for improved techniques for abnormality detection can be applied in connection with 3D medical images, specifically large volume 3D medical images obtained from whole-body scans or scans of larger body parts.

### Summary

The above need is met by the features of the independent claims. The features of the dependent claims define embodiments.

Hereinafter, techniques of detecting abnormalities represented in 3D medical image data will be disclosed. The 3D medical image data referred to in the following may correspond MRI image data or to CT image data. The 3D medical image data may be obtained from a whole-body scan of a patient, or from a scan of a larger body part of the patient, e.g., a scan of the complete head, a scan of the complete brain, or a scan of the thorax. However, other kinds of 3D medical image data could be considered as well.

In a first aspect, a computer-implemented method for enabling abnormality detection in 3D medical image data is provided. The method comprises receiving training input data comprising 3D medical image data and medical reports associated with the 3D medical image data. Further, the method comprises applying, by one or more computer processors, a trained language model for extracting anatomy phrases from the medical reports. Further, the method comprises training, by the one or more computer processors, a classification model based on the 3D medical image data and the anatomy phrases, whereby the classification model is trained to generate output data indicative of an abnormality represented in the 3D medical image data.

In an example, the method further comprises applying, by the one or more computer processors, a trained anatomy detection network, to generate, based on the anatomy phrases, an attention map for the 3D medical image data. The trained anatomy detection model may be based on a trained image segmentation network for segmenting the 3D medical image data based on the anatomy phrases. The attention map may highlight one or more anatomical locations in the 3D medical image data. Such anatomical locations may correspond to one or more image segments of the 3D medical image data. The training of the classification model can then be based on the 3D medical image data and the attention map.

In an example, the method further comprises applying, by the one or more computer processors, the trained language model for calculating a degree of similarity of the anatomy phrases to segment labels of an anatomic image database. An attention score of the attention map can then be defined by the degree of similarity of the anatomy phrases to the segment labels.

In an example, the method further comprises applying, by the one or more computer processors, the trained language model for tokenizing the one or more anatomy phrases and extracts the corresponding embeddings, and the training of the classification model comprises combining the embeddings to a feature sequence calculated by the classification model, in particular concatenating the embeddings to the feature sequence calculated by the classification model.

In an example, the method further comprises receiving runtime input data comprising 3D medical image data and applying the trained classification model for generating output data from the 3D medical image data of the runtime input data, the output data being indicative of an abnormality represented in the 3D medical image data of the runtime input data. The runtime input data may be without medical reports.

In a second aspect, a computer system is provided. The computer system comprises one or more computer processors configured to:
- receive training input data comprising 3D medical image data and medical reports associated with the 3D medical image data; and
- apply a trained language model for extracting anatomy phrases from the at least one medical report, and
- train a classification model based on the 3D medical image data and the anatomy phrases, whereby the classification model is trained to generate output data indicative of an abnormality represented in the 3D medical image data.

In an example, the one or more computer processors are further configured to:
- apply a trained anatomy detection network, to generate, based on the anatomy phrases, an attention map for the 3D medical image data. The attention map may highlight one or more anatomical locations in the 3D medical image data. Such anatomical locations may correspond to one or more image segments of the 3D medical image data, and
- train the classification model based on the 3D medical image data and the attention map to generate the output data indicative of an abnormality represented in the 3D medical image data.

In an example, the one or more computer processors are further configured to:
- apply the trained language model for calculating a degree of similarity of the anatomy phrases to segment labels of an anatomic image database, and
wherein an attention score of the attention map is defined by the degree of similarity of the anatomy phrases to the segment labels.

In an example, the one or more computer processors are further configured to:
- apply the trained language model for tokenizing the anatomy phrases and extracting the corresponding embeddings, and
- when training the classification model, combine the embeddings to a feature sequence calculated by the classification model, in particular concatenate the embeddings to the feature sequence calculated by the classification model.

In an example, the one or more computer processors are further configured to:
- receive runtime input data comprising 3D medical image data; and
- apply the trained classification model to generate output data from the 3D medical image data of the runtime input data, the output data being indicative of an abnormality represented in the 3D medical image data of the runtime input data. The runtime input data may be without medical reports.

In a third aspect, a computer-implemented method for abnormality detection in 3D medical image data is provided. The method comprises receiving runtime input data comprising 3D medical image data. Further, the method comprises applying, by one or more computer processors, a trained classification model for generating output data from the 3D medical image data of the runtime input data. The output data are indicative of an abnormality represented in the 3D medical image data of the runtime input data. The trained classification model is trained based on training 3D medical image data and based anatomy phrases extracted, by a trained language model, from medical reports associated with the training 3D medical image data. The runtime input data may be without medical reports.

In a fourth aspect, a computer system is provided. The computer system comprises one or more computer processors configured to:
- receive runtime input data comprising 3D medical image data; and
- apply a trained classification model for generating output data from the 3D medical image data of the runtime input data, the output data being indicative of an abnormality represented in the 3D medical image data of the runtime input data.

The trained classification model is trained based on training 3D medical image data and based anatomy phrases extracted, by a trained language model, from medical reports associated with the training 3D medical image data. The runtime input data may be without medical reports.

In a fifth aspect, a computer program product is provided. The computer program product comprises computer readable instructions which, when executed by one or more computer processors, cause the one or more computer processors to carry out a method of any of the above aspects or examples.

In the above aspects and examples, the classification model may be based on a state-space sequence model (SSM), combined with a convolutional neural network (CNN), e.g., according to a Mamba architecture. The medical reports may correspond to or comprise radiology reports. But other types of medical reports could be considered as well.

### Brief description of figures

Fig. 1 illustrates a flow chart for illustrating a method of enabling abnormality detection by training a classification model;
Fig. 2 illustrates a computer system for implementing the method of Fig. 1;
Fig. 3 illustrates a flow chart for illustrating a method of performing abnormality detection based on a trained classification model;
Fig. 4 illustrates a computer system for implementing the method of Fig. 3;
Fig. 5 illustrates an example of a training architecture that can be used in the method of Fig. 1;
Fig. 6 illustrates a further example of a training architecture that can be used in the method of Fig. 1;
Figs. 7 and 8 schematically illustrates an example of data processing using the architecture of Fig. 5; and
Fig. 9 schematically further illustrates an example data processing architecture of Fig. 5.
Fig. 10 schematically further illustrates an example data processing using the architecture of Fig. 6.
Figs. 11 and 12 further illustrate data processing blocks that may be used in the examples of Figs. 9 and 10.

### Detailed description

In the following the solution according to the invention is described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the system.

Furthermore, in the following the solution according to the invention is described with respect to methods and systems for use as well as with respect to methods and systems for training of a function (a machine learning algorithm, ML comprising, e.g., a neuronal network). Features, advantages or alternative embodiments herein can be assigned to the other claimed objects and vice versa. In other words, claims for methods and systems for training can be improved with features described or claimed in context of the methods and systems for use, and vice versa.

In particular, the trained (machine learning, ML) function (algorithm) of the methods and systems for use can be adapted by the methods and systems for training a function (ML algorithm, e.g., a neuronal network). Furthermore, the input data can comprise advantageous features and embodiments of the training input data, and vice versa. Furthermore, the output data can comprise advantageous features and embodiments of the output training data, and vice versa.

In general, a trained function mimics cognitive functions that humans associate with other human minds. In particular, by training based on training data the trained function is able to adapt to new circumstances and to detect and extrapolate patterns.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, a trained function can comprise a neural network, a support vector machine, a decision tree and/or a Bayesian network, and/or the trained function can be based on k-means clustering, Qlearning, genetic algorithms and/or association rules. In particular, a neural network can be a deep neural network (DNN) such as a convolutional neural network (CNN). Furthermore, a neural network can be an adversarial network, a deep adversarial network and/or a generative adversarial network.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

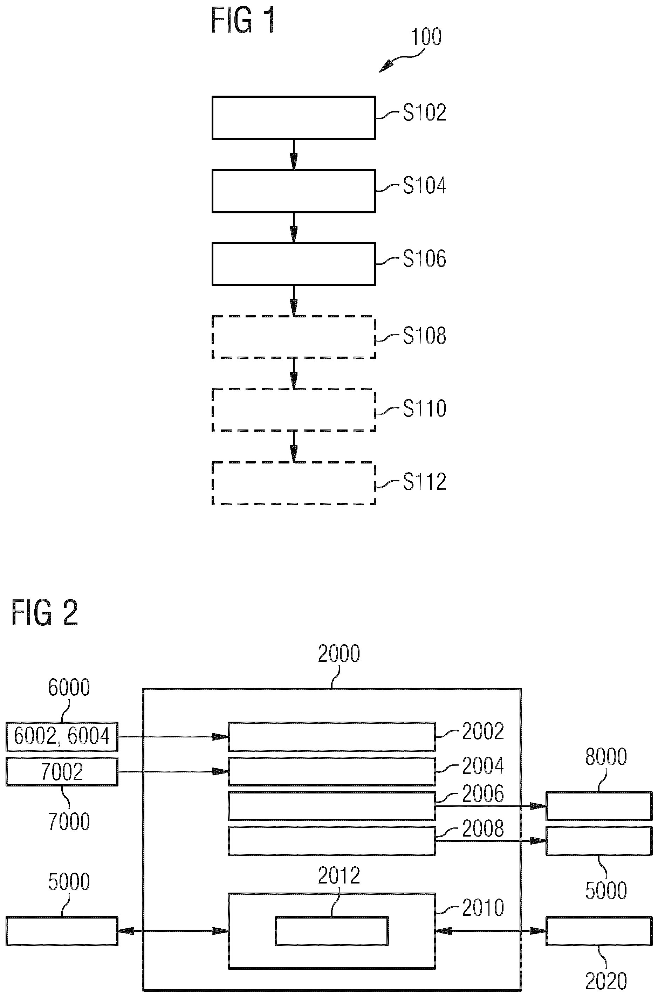

Fig. 1 shows a flow chart for illustrating a computer-implemented method 100 for enabling abnormality detection. The method 100 comprises steps S102 (receiving training input data), S104 (applying a trained language model), and S106 (training classification model). Optionally, the method can further comprise one or more of steps S108 (receiving runtime input data), S110 (applying the trained classification model), and S112 (outputting the trained classification model).

More specifically, the computer-implemented method 100 comprises, in step S102, receiving training input data. The training input data may be received by a computer interface. The training input data comprise 3D medical image data and medical reports associated with the 3D medical image data. For example, a dataset of the training input data could include a medical report based on 3D medical image data of a patient. The medical report can be a radiology report. The 3D medical image data may be obtained from a whole-body scan of a patient, or from a scan of a larger body part of the patient, e.g., a scan of the complete head, a scan of the complete brain, or a scan of the thorax. However, other kinds of 3D medical image data could be considered as well.

The method 100 further comprises, in step S104, applying a trained language model to medical reports, to extract anatomy phrases from the medical reports. Step S104 may be performed by one or more computer processors implementing the trained language model.

The method 100 further comprises, in step S106, training a classification model based on the 3D medical image data and the anatomy phrases. The classification model is trained to generate output data indicative of an abnormality represented in the 3D medical image data, e.g., diseased tissue, a lesion, an inflammation, or the like. The output data may also distinguish between different types of abnormality. In some scenarios, the output data may also indicate an anatomic location of the abnormality, e.g., in terms of an anatomic part or organ affected by the abnormality.

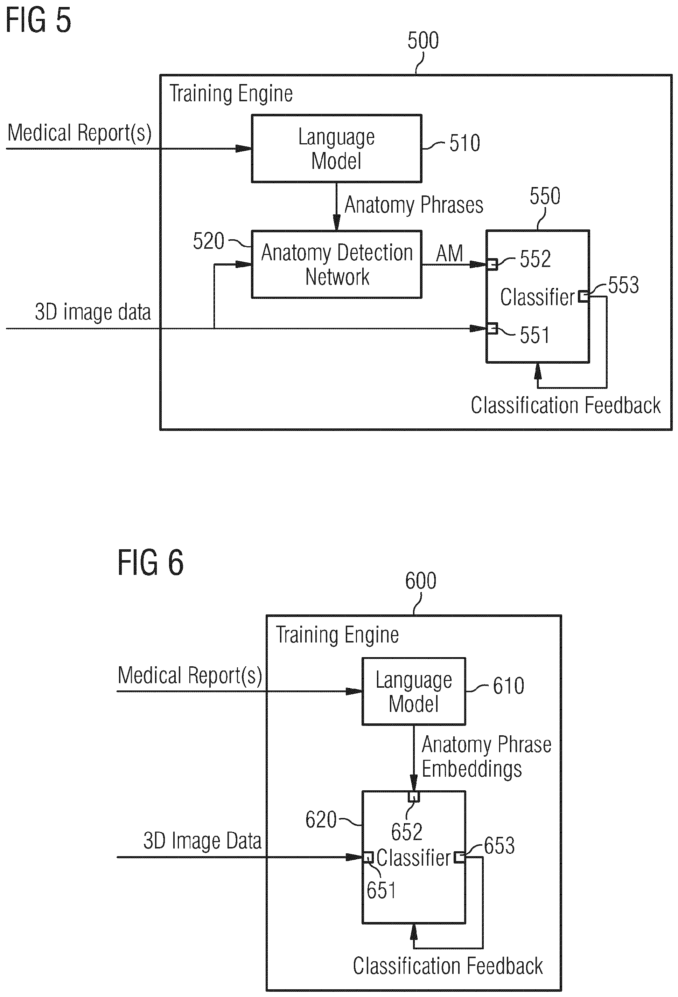

The training of step S106 may be based on an attention map that highlights one or more image segments of the 3D medical image data. A trained anatomy detection network may generate the attention map based on the anatomy phrases. The anatomy detection network may be based on a pre-trained image segmentation network coupled to an anatomic image database, e.g., an anatomy atlas. The trained language model may calculate a degree of similarity of the anatomy phrases to segment labels of the anatomic image database. An attention score of the attention map may be defined by the degree of similarity of the one or more anatomy phrases to the segment labels. Fig. 5 schematically illustrates an example of a corresponding training architecture.

Further, the training of step S106 could be based on applying the trained language model to tokenize the anatomy phrases and extract the corresponding embeddings. The embeddings can then be combined by a feature sequence calculated by the classification model, e.g., by concatenating the embeddings to the feature sequence. Fig. 6 schematically illustrates an example of a corresponding training architecture.

Optionally, the method can further comprise, at S108, receiving runtime input data. The runtime input data comprise 3D medical image data to be subjected to abnormality detection. The runtime input data may have a similar structure as the training input data, however without comprising the medical reports. The runtime input data may be received by a computer interface, e.g., the same computer interface as used for receiving the training input data, or a different computer interface. The method can then further comprise, at step S110, applying the trained classification model to generate output data indicative of an abnormality represented in the 3D medical image data of the runtime input data. For example, the output data could be indicative of diseased tissue, a lesion, an inflammation, or the like. The output data may also distinguish between different types of abnormality. In some scenarios, the output data may also indicate an anatomic location of the abnormality, e.g., in terms of an anatomic part or organ affected by the abnormality.

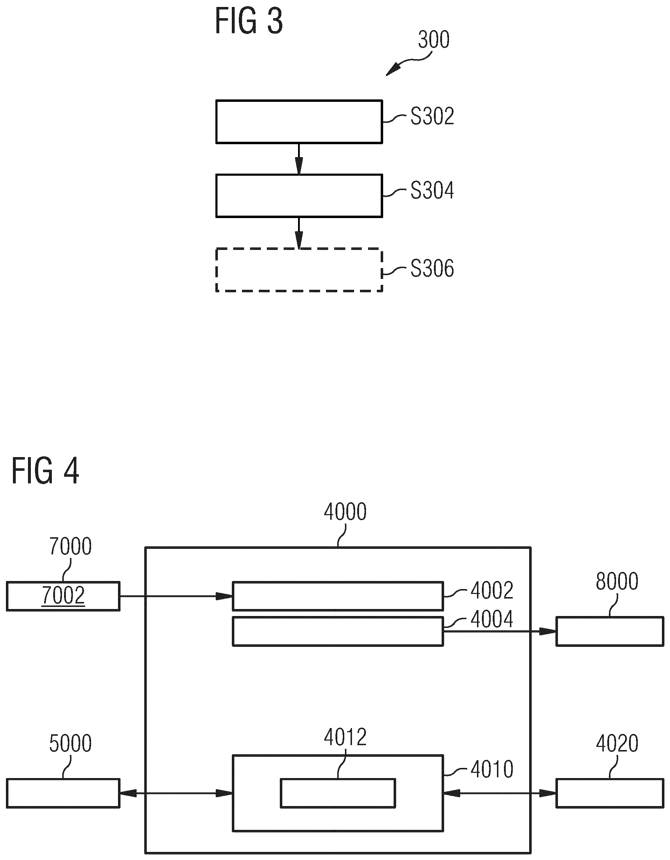

Optionally, the method can further comprise, at S112, outputting the trained classification model for use in abnormality detection on runtime input data, e.g., in a method as explained in connection with Fig. 3 or by a computer system as explained in connection with Fig. 4. The trained classification model can be output via a computer interface, e.g., the same computer interface as used for receiving the training input data, or a different computer interface.

Accordingly, in the method 100 of Fig. 1 the anatomy phrase(s) provided by the language model may be used as additional input for training the classification model. In this way, anatomy awareness training process can be achieved in an efficient manner, as the anatomy phrase(s) may correlate to anatomical locations of potential abnormalities.

Fig. 2 illustrates a computer system 2000 which may be used to implement the method 100 of Fig. 1.

As illustrated, the computer system 2000 may comprise a first computer interface 2002 configured for receiving the training input data 6000. As mentioned above, the training input data 6000 comprise 3D medical image data 6002 and medical reports 6004 associated with the 3D medical image data. The 3D medical image data 6002 may correspond MRI image data or to CT image data. The 3D medical image data may be obtained from a whole-body scan of a patient, or from a scan of a larger body part of the patient, e.g., a scan of the complete head, a scan of the complete brain, or a scan of the thorax. However, other kinds of 3D medical image data could be considered as well.

As further illustrated, the computer system 2000 may comprise a second computer interface 2004 configured for receiving the runtime input data 7000. As mentioned above, the runtime input data comprise 3D medical image data 7002 (but without medical reports). The 3D medical image data 7002 may be obtained from a whole-body scan of a patient, or from a scan of a larger body part of the patient, e.g., a scan of the complete head, a scan of the complete brain, or a scan of the thorax. However, other kinds of 3D medical image data could be considered as well.

As further illustrated, the computer system 2000 may comprise a third computer interface 2006 configured for outputting the output data 8000 generated by the trained classification model, i.e., output data 8000 indicative of an abnormality represented in the 3D medical image data 7002 of the runtime input data 7000. For example, the output data 8000 could be indicative of diseased tissue, a lesion, an inflammation, or the like. The output data 8000 may also distinguish between different types of abnormality. In some scenarios, the output data 8000 may also indicate an anatomic location of the abnormality, e.g., in terms of an anatomic part or organ affected by the abnormality.

As further illustrated, the computer system 2000 may comprise a fourth computer interface 2008 configured for outputting the trained classification model 5000.

As further illustrated, the computer system 2000 may comprise one or more computer processors 2012. The one or more computer processors 2012 may be part of a computation unit 2010. The one or more computer processors 2012 may be configured to apply the trained language model to extract the anatomy phrases from the medical reports, as explained in connection with step S104. Further, the one or more computer processors 2012 may be configured to train the classification model 5000 based on the 3D medical image data 6002 of the training input data 6000 and based on the anatomy phrases, as explained in connection with step S106. In some scenarios, the one or more computer processors 2012 may be further configured to apply the trained anatomy detection network to the 3D medical image data 6002, to generate, based on the anatomy phrases, the attention map used in training of the classification model 5000. In some scenarios, the one or more computer processors 2012 may be further configured to apply the trained classification model to the 3D medical image data 7002 of the runtime input data 7000, to generate the output data indicative of an abnormality represented in the 3D medical image data 7002 of the runtime input data 7000.

Operation of the computer processor(s) 2012 may be based on computer readable instructions 2020. The computer readable instructions 2020 may be provided as a computer program product, e.g., in the form of a computer-readable storage medium. The computer readable instructions 2020, when executed by the one or more computer processors 2012, may cause the computer system 2000 to carry out the method 100 of Fig. 1.

Fig. 3 shows a flow chart for illustrating a computer-implemented method 300 for abnormality detection using a trained classification model, e.g., as provided by the method of Fig. 1. The method 300 comprises steps S302 (receiving runtime input data) and S304 (applying the trained classification model). Optionally, the method can further comprise step S308 (outputting output data of the trained classification model).

More specifically, the computer-implemented method 300 comprises, in step S302, Optionally, the method can further comprise, at S108, receiving runtime input data. The runtime input data may be received by a computer interface. The runtime input data comprise 3D medical image data to be subjected to abnormality detection. The 3D medical image data may be obtained from a whole-body scan of a patient, or from a scan of a larger body part of the patient, e.g., a scan of the complete head, a scan of the complete brain, or a scan of the thorax. However, other kinds of 3D medical image data could be considered as well.

The method further comprises, at step S304, applying the trained classification model to generate output data indicative of an abnormality represented in the 3D medical image data of the runtime input data. For example, the output data could be indicative of diseased tissue, a lesion, an inflammation, or the like. The output data may also distinguish between different types of abnormality. In some scenarios, the output data may also indicate an anatomic location of the abnormality, e.g., in terms of an anatomic part or organ affected by the abnormality.

In the method 300, the trained classification model is trained based on training 3D medical image data and based anatomy phrases extracted, by a trained language model, from medical reports associated with the training 3D medical image data. For enabling this training, the trained classification model may be provided with an additional training input for receiving data derived from the anatomy phrases, e.g., the attention map derived from the anatomy phrases or the embeddings of the tokenized anatomy phrases.

The method can further comprise, at S306, outputting the output data of the trained classification model. The can be output via a computer interface, e.g., the same computer interface as used for receiving the runtime input data, or a different computer interface.

Accordingly, in the method 300 of Fig. 3 the classification model may be pre-trained in an anatomy aware manner, efficiently utilizing the medical reports as source of contextual anatomical information.

Fig. 4 illustrates a computer system 4000 which may be used to implement the method 400 of Fig. 3.

As illustrated, the computer system 4000 may comprise a first computer interface 4002 configured for receiving the runtime input data 7000. As mentioned above, the runtime input data 7000 comprise 3D medical image data 7002. The 3D medical image data 7002 may be obtained from a whole-body scan of a patient, or from a scan of a larger body part of the patient, e.g., a scan of the complete head, a scan of the complete brain, or a scan of the thorax. However, other kinds of 3D medical image data could be considered as well.

As further illustrated, the computer system 4000 may comprise a second computer interface 4004 configured for outputting the output data 8000 generated by the trained classification model, i.e., output data 8000 indicative of an abnormality represented in the 3D medical image data 7002 of the runtime input data 7000. For example, the output data 8000 could be indicative of diseased tissue, a lesion, an inflammation, or the like. The output data 8000 may also distinguish between different types of abnormality. In some scenarios, the output data 8000 may also indicate an anatomic location of the abnormality, e.g., in terms of an anatomic part or organ affected by the abnormality.

As further illustrated, the computer system 4000 may comprise one or more computer processors 4012. The one or more computer processors 4012 may be part of a computation unit 4010. The one or more computer processors 2012 may be configured to apply the trained classification model 5000 based on the 3D medical image data 7002 of the runtime input data 7000, to generate the output data indicative of an abnormality represented in the 3D medical image data 7002 of the runtime input data 7000.

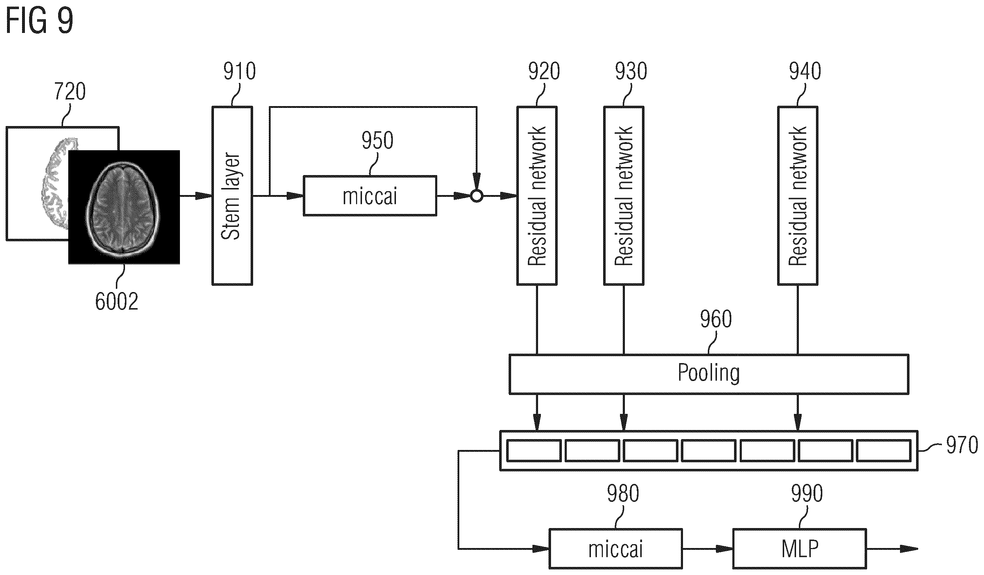

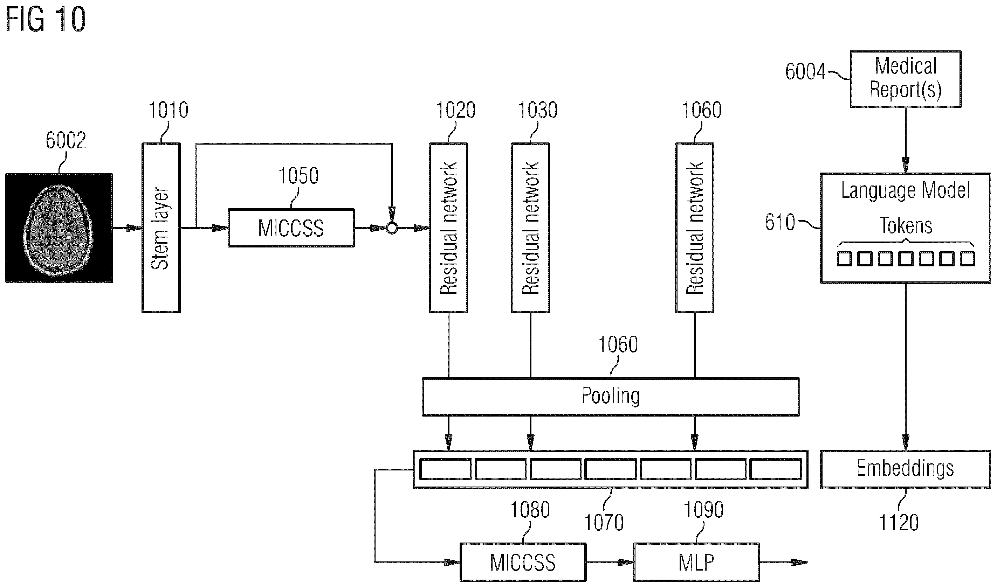

In the methods 100, 300, the classification model can be based on an SSM, combined with a CNN. As illustrated by Figs. 9 and 10, such combination may be implemented according to a Mamba architecture, e.g., nnMamba.

Operation of the computer processor(s) 4012 may be based on computer readable instructions 4020. The computer readable instructions 4020 may be provided as a computer program product, e.g., in the form of a computer-readable storage medium. The computer readable instructions 4020, when executed by the one or more computer processors 4012, may cause the computer system 4000 to carry out the method 300 of Fig. 3.

Fig. 5 schematically illustrates a first variant of an rachitecture 500 for implementing the training of the classication model. As illustrated, the architecture 500 comprises a language model 510, a trained anatomy detection network 520, and the classification model (classifier) 550. The annatomy detection network 520 may also be more shortly referred to as "anatomy detector". The language model 510 extracts the anatomy phrases from the medical reports. The anatomy detection network 520 utilizes the anatomy phrases as input for segmenting the 3D medical image data and generating an attention map (AM), or a set of multple attention maps, each corresponding to a different anatomic location, region, or structure.

The language model 510 can be a pre-trained Large Language Model (LLM). For example, the language model 510 can be based on the SBERT (Sentence BERT) model, see "Sentence-BERT: Sentence Embeddings using Siamese BERT-Networks", by Reimers, N. and Gurevych I., Proceedings of the 2019 Conference on Empirical Methods in Natural Language Processing and the 9th International Joint Conference on Natural Language Processing (EMNLP-IJCNLP), pages 3982-3992, Hong Kong, China. Association for Computational Linguistics (2019). The language model 510 receives the medical reports 6004 of the training input data and extracts anatomy phrases, which are associated with anatomic locations, from the medical reports. The language model 510 may calculate a degree of similarity of the one or more anatomy phrases to segment labels of an anatomic image database, e.g., an anatomy atlas. Such anatomic image database may be integrated within the training architecture 500, or the anatomic image database could be coupled via a data network, e.g., the Internet, to the language model 510 and/or the anatomy detection network 520. An attention score of the attention map may be defined by the degree of similarity of the one or more anatomy phrases to the segment labels.

The anatomy detection network 520 can be based on a pre-trained image segmentation network, such as the SynthSeg network, see "SynthSeg: Segmentation of brain MRI scans of any contrast and resolution without retraining", by Billot, B. et al, Medical Image Analysis (2023). The attention map may highlight one or more anatomical locations, structures, or regions in the 3D medical image dat. Such anatomical location, structure, or region may correspond to one or more image segments of the 3D medical image data. The classification model 550 can then use the attention map as further training input.

The language model 510 may calculate a degree of similarity of the one or more anatomy phrases to segment labels of an anatomic image database, e.g., an anatomy atlas. An attention score of the attention map may be defined by the degree of similarity of the one or more anatomy phrases to the segment labels. Here, hard segmentation could be implemented by using a binary attention score, corresponding to the level of similarity being either above or below a threshold. A soft segmentation could be implemented by considering one or more intermediate levels of similarity.

As illustrated in Fig. 5, training of the classification model 550 is based on a first training input 551, which receives the 3D image data, a second training input 552, which receives the attention map(s). A training output 553 provides output data indicative of one or more abnormalities as classification feedback. The classification feedback may be used to compare the output of the classification model 550 with a nominal output and minimize the corresponding difference, e.g., by an iterative optimization minimizing a loss function correspondingly defined. The nominal output can be based on expert annotations, e.g., indicating presence or type of the abnormality present in the 3D medical image data. During runtime usage of the trained classification model 550, the first training input is used for receiving the 3D image data of the runtime input data, and the training output 553 is used for providing the output data indicative of an abnormality represented in the 3D medical image data of the runtime input data. During runtime operation, the second training input 552 is left idle.

Fig. 6 schematically illustrates a second variant of an ML model 600 for implementing the trained function. As illustrated, the ML model 600 comprises a language model 610 and a classification model (classifier) 650. The language model 610 can be a pre-trained LLM or Small Language Model (SLM), such as a model of the Phi-3 model family, see "New models added to the Phi-3 family, available on Microsoft Azure", by Bilenko M., online, https://azure.microsoft.com/en-us/blog/new-models-added-to-the-phi-3-family-available-on-microsoft-azure/ (2024). In the second variants, output of the language model 610 is fed directly to the classification model 650. For this purpose, the language model 610 tokenizes the anatomy phrases and extracts the corresponding embeddings, e.g., in terms of sentence embeddings. The embeddings are then concatenated (or otherwise combined) to a feature sequence calculated by the classification model 650.

As illustrated in Fig. 6, training of the classification model 550 is based on a first training input 651, which receives the 3D image data, a second training input 652, which receives the output of the language model 610, i.e., the embeddings of the tokenized anatomy phrases. A training output 653 provides output data indicative of one or more abnormalities as classification feedback. The classification feedback may be used to compare the output of the classification model 550 with a nominal output and minimize the corresponding difference, e.g., by an iterative optimization minimizing a loss function correspondingly defined. The nominal output can be based on expert annotations, e.g., indicating presence or type of the abnormality present in the 3D medical image data. During runtime usage of the trained classification model 650, the first training input is used for receiving the 3D image data of the runtime input data, and the training output 653 is used for providing the output data indicative of an abnormality represented in the 3D medical image data of the runtime input data. During runtime operation, the second training input 652 is left idle.

In both the first variant and the second variant, the classification model 550, 650 can be based on an SSM, combined with a CNN. As illustrated by Figs. 9 and 10, such combination may be implemented according to a Mamba architecture, e.g., nnMamba.

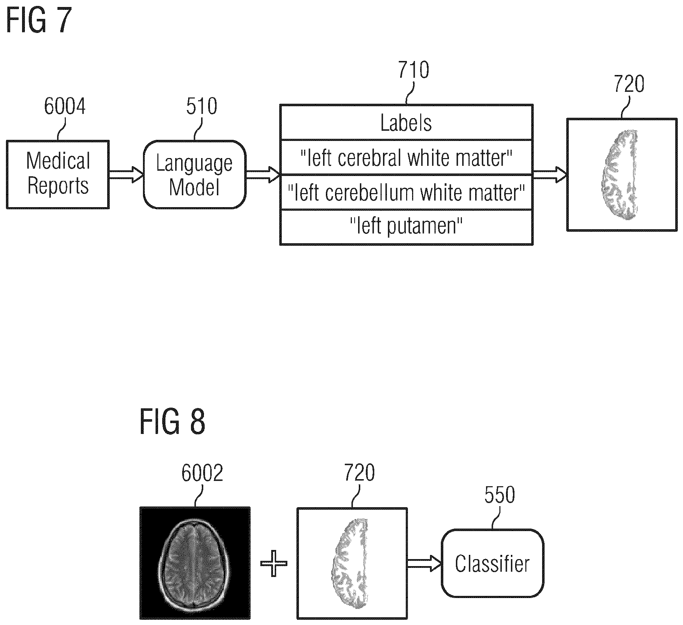

Figs. 7 and 8 schematically illustrate an example of data processing in the first variant. As illustrated in Fig. 7, the medical reports 6004 are provided to the language model 510, which extracts anatomy phrases from the medical reports. To generate attention maps corresponding to the anatomy phrases, similar phrases are fetched from the anatomy atlas, e.g., a brain MRI atlas. The language model may calculate the similarity of different phrases, and the antomy phrases may be ranked according to the calculated level of similarity. As a result, a set of N segmentation labels having the highest level of similarity is obtained. In the example of Fig. 7, it is assumed that three segmentation with similarity to the extracted anatomy phrases are found, nameley "left cerebral white matter", "left cerebellum white matter", and "left putamen". These segmentation labels are used to segment the 3D image data accordingly, by identfying image segments corresponding to these labels. These image segments are used to construct the attention map 720, which highlights the anatomic structure corresponding to the segmentation labels.

As ilsustrated in Fig. 8, the attention map 720 is added as a second an additional training input to the 3D image data 6002. During the training process, the classification model 550 may incorporate the attention map 720 by adjusting weights corresponding to pixels or voxels of the 3D medical image data 6002. Various kinds of attention-based learning mechansims may be applied.

Fig. 9 further illustrates data processing by the classification model 550 of the first variant, assuming an example implementation based on an nnMamba architecture. As illustrated, the classification model 550 is based on a CNN architecture having a stem layer 910 and residual networks (ResNets) 920, 930, 940. The residual networks 920, 930, 940 help to reduce dimensionality an enable efficient capturing of the long-range relations in the 3D medical images. Further, multi-resolution characteristics may be extracted from the 3D medical images, so that the classification model 550 can efficiently learn from different resolutions. A block 950 denoted as "MICCSS" is provided for combining the CNN layers with Mamba layers. In the example of Fig. 9, output of the residual networks 920, 930, 940 is provided to a pooling stage 960, which applies average global pooling (or some other type of pooling) to generate a feature sequence 970. The feature sequence 970 is provided to a further MICCSS block 980 and to a Multilayer-Perceptron (MLP) block 990 to provide the output data.

Fig. 10 further illustrates data processing by the classification model 650 of the second variant, assuming an example implementation based on an nnMamba architecture. As illustrated, the classification model 650 is based on a CNN architecture having a stem layer 1010 and residual networks (ResNets) 1020, 1030, 1040. The residual networks 1020, 1030, 1040 help to reduce dimensionality an enable efficient capturing of the long-range relations in the 3D medical images. Further, multi-resolution characteristics may be extracted from the 3D medical images, so that the classification model 650 can efficiently learn from different resolutions. A block 1050 denoted as "MICCSS" is provided for combining the CNN layers with Mamba layers. In the example of Fig. 10, output of the residual networks 1020, 1030, 1040 is provided to a pooling stage 1060, which applies average global pooling (or some other type of pooling) to generate a feature sequence 1070. The output of the language model 610, in paricualar the sentence embeddings 1120 of the tokenized anatomy phrases are concatendated to the feature sequence 1070. The resulting combination of the feature sequence 1070 and the sentence embeddings 1120 is provided to a further MICCSS block 1080 and to a MLP block 1090 to provide the output data.

In the second variant, instead of incorporating the medical reports by an attention map, the medical reports are involved linguistically. In particular, the embeddings extracted from language model provide an additional feature sequence which is combined to the feature sequence calculated from the 3D medical image data.

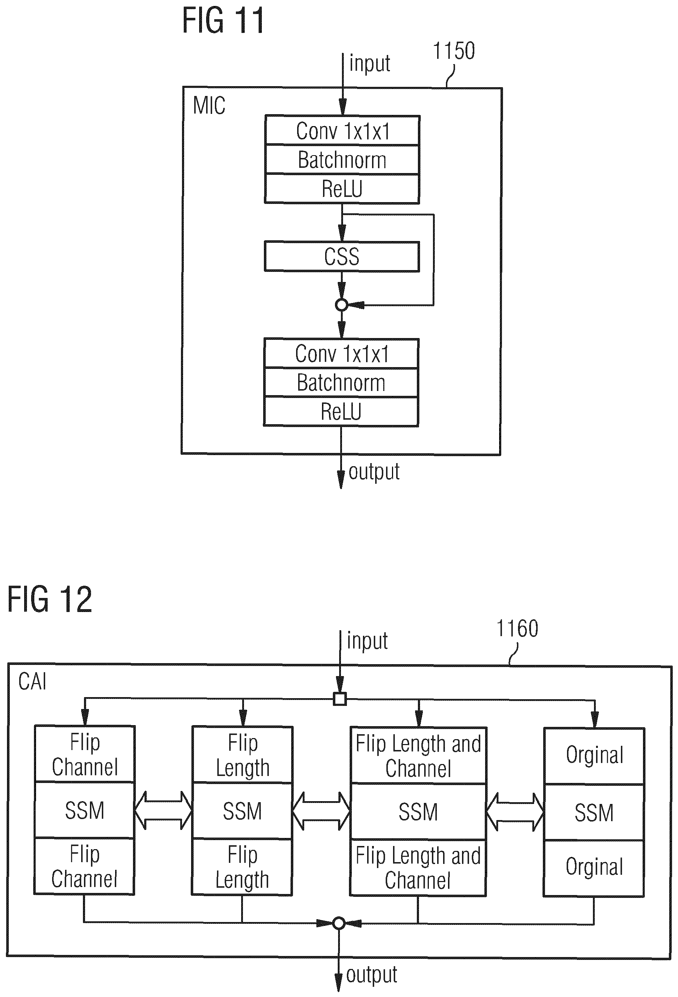

Figs. 11 and 12 further illustrate an example implementation of the MICCSS blocks 950, 980, 1050, 1080. As illustrated by Fig. 11, the MICCSS blocks 950, 980, 1050, 1080 may be formed by a MIC (Mamba-In-Comvolution) block 1150, which incorporates a CSS (Channel-Spatial Siamese) inputting block 1160. Details of the CSS block 1160 are shown in Fig. 12. The MIC block 1150 integrates SSMs with convolutional layers, which include an 1×1×1 convolution layer, a batch normalization (Batchnorm) layer, and a rectified linear unit (ReLU) activation layer. The CSS inputting block 1160 provides processing of the data by multiple SSMs with shared weights. In Fig. 12, the sharing of weights by the SSMs is illsutrated by horizontal block arrows.

Further details concerning the utilized nnMamba architecture and its elements can be found in "nnMamba: 3D Biomedical Image Segmentation, Classification and Landmark Detection with State Space Model", by Gong, H., et al., http://arxiv.org/abs/2402.03526 (2024).

It was found that both the first variant and the second variant can be used to efficiently enable anatomy context-aware abnormality detection. Choice between using the first variant and the second variant may depend on the intended application and available resources. If radiology reports (or other medical reports) and anatomy phrases are abundant, the second variant may be preferable. Otherwise, the first variant may be preferable as the correlation between the attention maps and disease locations may be higher than the correlation between the embeddings of the anatomy phrases and disease locations, which means that the first variant may provice higher accuracy.

For evaluation, performance of abnormality detectors based on the first variant and the second variant were compared to known abnormality detectors, considering a test dataset of 3D brain MRI data. The test data set was divided into two classes, denoted as set A and set B. Set A consisted by datasets with the following disease abnormalities: demyelination, lesion, and focal dysplasia. Datasets with other types of disease abnormalities where assigned to Set B. The distribution of the datasets for training, validation, and classification test were for Set A:341, 114 and 103, respectively, and for set B: 73, 26 and 45, respectively. Evaluation results of the trained networks can be seen below in Table 1.

**Table 1**

| | **Accuracy** | **F1 macro** |
|---|---|---|
| **Baseline** | 0.814 | 0.449 |
| **nnMamba Variant 1 (hard segmentation)** | 0.814 | 0.724 |
| **nnMamba Variant 1 (soft segmentation)** | 0.821 | 0.750 |
| **nnMamba Variant 2** | 0.786 | 0.635 |

In Table 1, a scenario denoted as "baseline" represents a comparative benchmark where all the predictions of the trained classification model are in favor of the majority class.

When experimenting with various known types of classification model, based on different CNN architectures, it was found that the prediction performance was around the baseline or even fell behind the baseline scenario. Further, it was found that these known types of classification model were computationally costly. A classification model trained in accordance with the illustrated concepts were tested in three versions: The first variant in connection with the nnMamba architecture of Fig. 9 and hard segmentation of the attention map, the first variant in connection with the nnMamba architecture of Fig. 9 and soft segmentation of the attention map, and the second variant based on using the Phi-3 LLM. As can be seen from Table 1, in each case the illustrated concepts resulted in accuracy equal to or even in excess of the baseline and also improved the F1 macro score, which is a criterion for performance assessment, significantly.

As can be seen, concepts of the present disclosure may be used to teach a deep neural network where the anatomical objects are located. This may in turn enable the efficient and accurate classification of abormalities in the 3D medical image data. The concepts may be used for efficiently handling the large computational demands associated with processing of large volume 3D medical image data scans and also for managing inherent complexity of the underlying datasets. For example, between such datasets, abnormality structures may vary widely in size and detail, e.g., i.e., from very small lesions to large and widespread abnormalities. Further, types of abnormalities may vary and available training data may represent such abnormality types in an imbalanced manner. In the illustrated concepts, such challenges may be efficiently addressed by uitilizing the medical reports as source of contextual information.

In the illustrated concepts, the Mamba architecture, e.g., nnMamba, may be applied within the classification network, which helps to ensure that computational complexity scales linearly with the number of pixels in the volume covered by the 3D medical image data. The Mamba architecture may be combined with convolutional layers to enable the classification model to effectively learn both long-range dependencies and local anatomical details. The illustrated concepts may thus also be suitable for application to 3D medical image data in the form of high-resolution 3D brain MRIs.

The trained classification model of the illustrated concepts can leverage the guidance of the medical reports for improved abnormality detection, also considering cases where disease abnormalities are confined to small pixel regions or cases where disease abnormalities are spread across larger areas, which may in turn vary from patient to patient. The illustrated concepts may help to ensure that performance of abnormality detection remains high, even if the number and diversity of the considered patients increases. The latter may be especially valuable clinical application. By using the anatomy phrases extracted from the medical reports as contextual information, the classification model can learn to focus on the relevant areas where the abnormality is very likely to be present. This may in turn help the trained classification model to detect the abnormality, even if the considered patient's anatomy is different from the anatomies underlying the 3D image data used in training.

### List of Reference

- 100:: method for enabling abnormality detection
- S102:: receiving training input data
- S104:: applying language-based model
- S106:: training classification model
- S108:: receiving runtime input data
- S110:: applying trained classification model
- S112:: outputting trained classification model

- 2000:: system for enabling abnormality detection
- 2002:: first computer interface
- 2004:: second computer interface
- 2006:: third computer interface
- 2008:: fourth computer interface
- 2010:: computation unit
- 2012:: computer processor(s)
- 2020:: computer readable instructions
- 5000:: classification model
- 6000:: training input data
- 6002:: 3D medical image data
- 6004:: medical reports
- 7000:: runtime input data
- 7002:: 3D medical image data
- 8000:: output data

- 300:: a method for abnormality detection
- S302:: receiving runtime input data
- S304:: applying trained classification model
- S306:: outputting output data

- 4000:: computer system
- 4002:: first computer interface
- 4004:: second computer interface
- 4010:: computation unit
- 4012:: computer processor(s)
- 4020:: computer readable instructions

- 500:: training architecture
- 510:: language model
- 520:: anatomy detection network
- 550:: classification model
- 551:: first training input
- 552:: second training input
- 553:: training output

- 600:: training architecture
- 610:: language model
- 650:: classification model
- 651:: first training input
- 652:: second training input
- 653:: training output

- 710:: labels
- 720:: attention map

- 910:: stem layer
- 920, 930, 940:: residual network layers
- 950:: MICCSS block
- 960:: pooling
- 970:: feature sequence
- 980:: MICCSS block
- 990:: MLP block

- 1010:: stem layer
- 1020, 1030, 1040:: residual network layers
- 1050:: MICCSS block
- 1060:: pooling
- 1070:: feature sequence
- 1080:: MICSS block
- 1090:: MLP block
- 1120:: embeddings
- 1150:: MIC block
- 1160:: CSS block

## Claims

1. A computer-implemented method (100) for enabling abnormality detection in three-dimensional, 3D, medical image data, comprising:
- receiving (S102) training input data (6000) comprising 3D medical image data (6002) and medical reports (6004) associated with the 3D medical image data (6002),
- applying (104), by one or more computer processors (2008), a trained language model (510; 610) for extracting anatomy phrases from the medical reports (6004), and
- training (S106), by the one or more computer processors (2008), a classification model (550; 650) based on the 3D medical image data (6002) and the anatomy phrases, whereby the classification model (550; 650) is trained to generate output data (8000) indicative of an abnormality represented in the 3D medical image data (6002).

2. The method of claim 1, comprising:
- applying, by the one or more computer processors (2008), a trained anatomy detection network (520) for segmenting the 3D medical image data based on the anatomy phrases, to generate, based on the anatomy phrases, an attention (720) map for the 3D medical image data (6002),
wherein said training (S106) of the classification model (550; 650) is based on the 3D medical image data (6002) and the attention map (720).

3. The method of claim 2, comprising:
- applying, by the one or more computer processors (2008), the trained language model (510) for calculating a degree of similarity of the anatomy phrases to segment labels (710) of an anatomic image database, and
wherein an attention score of the attention map (720) is defined by the degree of similarity of the anatomy phrases to the segment labels (720).

4. The method of claim 1, comprising:
- applying, by the one or more computer processors (2008), the trained language model (610) for tokenizing the one or more anatomy phrases and extracts corresponding embeddings (1120), and
wherein said training (S106) of the classification model (550; 650) comprises combining the embeddings (1120) to a feature sequence (1070) calculated by the classification model (650).

5. The method of claim 4,
wherein said training (S106) of the classification model (550; 650) comprises concatenating the embeddings (1120) to the feature sequence (1070) calculated by the classification model (650).

6. The method of any of the preceding claims,
wherein the classification model (550; 650) is based on a state-space sequence model, SSM, combined with a convolutional neural network, CNN.

7. The method of any of the preceding claims, comprising:
- receiving (S108) runtime input data (7000) comprising 3D medical image data (7002); and
- applying (S110) the trained classification model (550; 650) for generating output data (8000) from the 3D medical image data of the runtime input data (7000), the output data (8000) being indicative of an abnormality represented in the 3D medical image data (7002) of the runtime input data (7000).

8. A computer system (2000), comprising:
one or more computer processors (2012) configured to:
- receive training input data (6000) comprising 3D medical image data (6002) and medical reports (6004) associated with the 3D medical image data (6002); and
- apply a trained language model (510; 610) for extracting anatomy phrases from the medical reports (6004), and
- train a classification model (550; 650) based on the 3D medical image data (6002) and the anatomy phrases (6004), whereby the classification model (550; 650) is trained to generate output data (8000) indicative of an abnormality represented in the 3D medical image data (6002).

9. The computer system (2000) of claim 8,
wherein the one or more computer processors (2012) are further configured to:
- apply a trained segmentation network (520) for segmenting the 3D medical image data (6002) based on the anatomy phrases, to generate, based on the anatomy phrases, an attention map (720) for the 3D medical image data (6002), and
- train the classification model (550; 650) based on the 3D medical image data (6002) and the attention map (720) to generate the output data (8000) indicative of an abnormality represented in the 3D medical image data (6002).

10. The computer system (2000) of claim 9,
wherein the one or more computer processors (2012) are further configured to:
- apply the trained language model (510) for calculating a degree of similarity of the anatomy phrases to segment labels (710) of an anatomic image database, and
wherein an attention score of the attention map (720) is defined by the degree of similarity of the anatomy phrases to the segment labels (710).

11. The computer system (2000) of claim 8,
wherein the one or more computer processors (2012) are further configured to:
- apply the trained language model (610) for tokenizing the anatomy phrases and extracting corresponding embeddings (1120), and
- when training the classification model (550; 650), combine the embeddings (1120) to a feature sequence (1170) calculated by the classification model (650).

12. The computer system (2000) of claim 11,
wherein the one or more computer processors (2012) are further configured to:
- when training the classification model (550; 650), concatenating the embeddings to the feature sequence calculated by the classification model (650).

13. The computer system (2000) of any of claims 8 to 12,
wherein the classification model is based on a state-space sequence model, SSM, combined with a convolutional neural network, CNN.

14. The computer system (2000) of any of claims 8 to 13,
wherein the one or more computer processors (2012) are further configured to:
- receive runtime input data (7000) comprising 3D medical image data (7002); and
- apply the trained classification model (550; 650) to generate output data (8000) from the 3D medical image data (7002) of the runtime input data (7000), the output data (8000) being indicative of an abnormality represented in the 3D medical image data (7002) of the runtime input data (7000).

15. A computer-implemented method (300) for abnormality detection in three-dimensional, 3D, medical image data, comprising:
- receiving (S302) runtime input data (7000) comprising 3D medical image data (7002); and
- applying (S304), by one or more computer processors (4008), a trained classification model (550; 650) for generating output data (80009 from the 3D medical image data (7002) of the runtime input data (7000), the output data being indicative of an abnormality represented in the 3D medical image data (7002) of the runtime input data (7000), wherein the trained classification model (550; 650) is trained based on training 3D medical image data (6000) and based on anatomy phrases extracted, by a trained language model (510; 610), from medical reports (6004) associated with the training 3D medical image data (6002).

16. A computer system (4000), comprising:
one or more computer processors (4012) configured to:
- receive runtime input data (7000) comprising 3D medical image data (7002); and
- apply a trained classification model (550; 650) for generating output data (8000) from the 3D medical image data (7002) of the runtime input data (7000), the output data (8000) being indicative of an abnormality represented in the 3D medical image data (7002) of the runtime input data (7000),
wherein the trained classification model (550; 650) is trained based on training 3D medical image data (6002) and based anatomy phrases extracted, by a trained language model (510; 610), from medical reports (6004) associated with the training 3D medical image data (6002).

17. A computer program product comprising computer readable instructions which, when executed by one or more computer processors (2012; 4012), cause the one or more computer processors (2012; 4012) to carry out the method of one of claims 1 to 7 or 15.
